(19) Europäisches Patentamt
European Patent Office
Office européen des brevets

(11) Publication number: 0 142 754
A2

(12) # EUROPEAN PATENT APPLICATION

(21) Application number: 84113194.9

(22) Date of filing: 02.11.84

(51) Int. Cl.[4]: C 07 D 233/60
A 61 K 31/415

(30) Priority: 04.11.83 ES 527268
25.06.84 ES 534124

(43) Date of publication of application:
29.05.85 Bulletin 85/22

(84) Designated Contracting States:
BE CH DE FR GB IT LI NL

(71) Applicant: FERRER INTERNACIONAL, S.A.
Gran Via Carlos III no 94
Barcelona 28(ES)

(72) Inventor: Foguet, Rafael
Llusanés 8
ES-08028 Barcelona(ES)

(72) Inventor: Forné, Ernesto, Dr.
Felipe de Paz 15
ES-08028 Barcelona(ES)

(72) Inventor: Ortiz, José A., Dr.
Córcega 429
ES-08037 Barcelona(ES)

(72) Inventor: Sacristán, Aurelio
Santa Amelia 2
ES-08034 Barcelona(ES)

(74) Representative: Kinzebach, Werner, Dr.
Patentanwälte Reitstötter, Kinzebach und Partner
Postfach 780
D-8000 München 43(DE)

(54) **2-Substituted-benzoic acid imidazoles, process for preparing them and pharmaceutical compositions containing them.**

(57) The invention relates to 2-substituted-benzoic acid imidazoles, a process for preparing them and pharmaceutical composition containing them. The compounds of this invention are platelet antiaggregating agents and are therefore useful in the treatment of cardiovascular diseases, respiratory disorders and inflammatory conditions. Furthermore, these compounds reduce metastasis in a large number of tumors.

EP 0 142 754 A2

Croydon Printing Company Ltd.

The invention relates to 2-substituted-benzoic acid imidazoles and their non-toxic addition salts, the process for preparing them and pharmaceutical compositions containing them.

The compounds of the invention have the general formula (I):

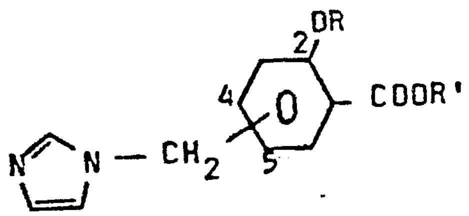

(I)

wherein

R and R' independently may be a hydrogen atom or a straight chain or branched $C_1$-$C_6$ alkyl group, and the imidazolylmethyl group is attached to the 4- or 5-position of the benzene ring,

and the non-toxic addition salts thereof.

Preferably R and R' represent a $C_1$-$C_4$ alkyl group, especially preferred a methyl group or a hydrogen atom.

The process for preparing the compounds according to the invention is characterized in that

A) a compound of the general formula (II)

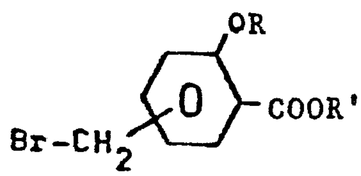

(II)

wherein R and R' have the meanings as defined above and the bromomethyl group is attached to the 4- or 5-position of the benzene ring, is reacted with imidazole in an aprotic solvent in the presence of a strong base,

B) if desired, the so obtained ester of the general formula (Ia)

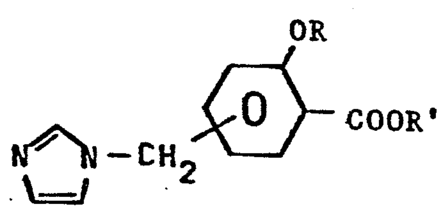

(Ia)

is saponified, and

C) if desired, the so obtained compound of the general formula (Ib)

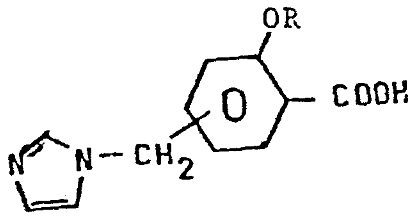

(Ib)

is subjected to an ether cleavage reaction, and

D) if desired, any of the compounds obtained above is converted to a non-toxic addition salt thereof.

The starting reagents alkyl 4- and 5-bromomethyl-2-alkoxybenzoates (II), may be prepared according to conventional methods disclosed in the literature by conveniently performing a bromination of their precursors, 4- und 5-alkyl-2-alkoxybenzoates, with N-bromosuccinimide in carbon tetrachloride.

The reaction of the starting reagents (II) with imidazole (step A) is carried out in the presence of a strong base, preferably sodium hydride, which enables to ionize the imidazole. An aprotic solvent is used for this reaction step, preferred is N,N-dimethylformamide. The reaction temperature may range from room temperature to the boiling temperature of the medium. Work up of the reaction mixture occurs via removal of the solvent under vacuum, addition of water and extraction with an inert solvent, preferably a chlorinated aliphatic hydrocarbon such as methylene chloride. The residue of the solvent extraction may be purified by crystallization or chromatography.

The so obtained esters may be converted to the corresponding carboxylic acids of the general formula (Ib) by saponification with an alkaline or alkaline earth hydroxide in an alcohol or aqueous alcohol. A suitable reaction medium is for instance methanol, ethanol, propanol or a mixture thereof or a mixture of said alcohol with water. The reaction temperature may range from room temperature up to the boiling point of the reaction medium. Conveniently the reaction is carried out at the boiling point of the reaction medium.
After the reaction the solvent is evaporated under vacuum. Addition of water, extraction with a non-polar solvent, preferably diethyl ether or tetrahydrofuran, acidification of the aqueous phase and finally purification of the formed crystals by recrystallization or washing with water yields the desired products.

If those compounds of the formula (I) are desired in which R and R' are hydrogen the compounds of the formula Ib are subjected to an ether cleavage reaction. Preferably this reaction is carried out with boron tribromide in a chlorinated aliphatic hydrocarbon solvent, preferably in methylene chloride. The temperature for the reaction with boron tribromide may range from room temperature down to -100°C. The so obtained precipitate is purified by crystallization to obtain the compounds of the general formula (I), wherein R and R' are hydrogen, in the form of the hydrobromide. If the product in free form is desired, it is reacted with an alkaline or alkaline earth hydroxide, such as sodium hydroxide or potassium hydroxide, in an aqueous medium. The reaction mixture is then extracted with a non-polar solvent, preferably diethyl ether or tetrahydrofuran, and the aqueous phase finally acidified to yield the desired product in free form.

If desired, the compounds of the general formula (I) in free form may be converted to the corresponding addition salts. Suitable reagents for this reaction are inorganic acids, such as sulphuric acid, phosphoric acid, hydrogen chloride and the like, organic acids, such as acetic acid, tartaric acid, citronic acid, lactic acid and the like and amines, like monoalkanolamines, dialkanolamines, and the like.

The compounds of this invention are platelet antiaggregating agents thus exhibiting a specific inhibitory effect on thromboxane synthetase, which makes them therapeutically useful in the treatment of cardiovascular diseases, such as thrombosis, arteriosclerosis, coronary contractions, arrhythmia, ischaemic cerebral attack, migraine, myocardial infarction, angina pectoris and hypertension; respiratory disorders, e.g. asthma and apnea; and inflammatory conditions of organs and limbs. In addition, these compounds, due to their inhibitory action on thromboxane synthetase, reduce metastasis in a large number of tumors.

The compounds of this invention mixed with pharmaceutically acceptable carriers can be administered by the oral route in the form of tablets, capsules, coated tablets, syrup, solution, etc., by injectable route and by rectal route at daily dosis ranging from 0,01 to 200 mg/kg.

A number of exmples will now be described in non-limitative manner to illustrate the invention.

## Example 1

### Methyl 4-bromomethyl-2-methoxybenzoate (II, 4 position)

A mixture containing 36,04 g of methyl 4-methyl-2-methoxybenzoate (B.P. 105-108°C/1,5 torrs and $n_D^{20}$ = 1,5352), 35,6 g of N-bromosuccinimide and 0,5 g of benzoyl peroxide in 200 ml of carbon tetrachloride is refluxed for 3 hours. Then the mixture is cooled, the succinimide is filtered off, the solvent is evaporated to dryness, and the resinous residue is distilled under vacuum to give 31 g (yield: 60%) of a colorless liquid (B.P. 124-128°C/ 0,2 torrs) which crystallizes on standing. M.P. 40-44°C.

Elemental analysis as C, H, Br correct.

IR Spectrum (KBr), $cm^{-1}$: 1700, 1300, 1250, 1210, 1090.

## Example 2

### Methyl-4-(1-imidazolylmethyl)-2-methoxybenzoate (I, R=R'= $CH_3$, 4 position)

To 2,86 g of sodium hydride in 270 ml of dry N,N-dimethylformamide are added dropwise 8,59 g of imidazole in 25 ml of N,N-dimethylformamide and the mixture is heated to 90°C for 1 hour. Then it is cooled and 31,0 g of methyl 4-bromomethyl-2-methoxybenzoate in 35 ml of N,N-dimethylformamide over a period of 45 minutes are added. The mixture is kept at 90°C for 1 hour, then cooled and the solvent is removed under vacuum. The residue is treated with water and extracted several

times with methylene chloride, and the residual semisolid is recrystallized from ethyl acetate to give 16,4 g (yield: 56 %) of a solid with M.P. 110-113°C and analysis correct.

The compound under constant melting point (116-118°C) is obtained by silicagel column chromatography; eluent, chloroform : methanol (99 : 1).

IR Spectrum (KBr), $cm^{-1}$: 3100, 3000-2800, 1700, 1420, 1290, 1250, 1080.

## E x a m p l e 3

### 4-(1-Imidazolylmethyl)-2-methoxybenzoic acid (I, R= $CH_3$, R'= H, 4 position)

To a solution of 11 g of methyl 4-(1-imidazolylmethyl)-2-methoxybenzoate in 90 ml of ethanol is added 28,9 ml of 2N potassium hydroxide and the mixture is refluxed for 2 hours. The ethanol is evaporated, 35 ml of water are added and the mixture is then extracted with ether; the aqueous phase is acidified with glacial acetic acid, and a white solid crystallizes upon cooling. The residue is filtered off and recrystallized from ethanol to give 8,09 g (yield: 76 %) of a white solid. M.P.: 195-197°C and analysis correct.

IR Spectrum (KBr), $cm^{-1}$: 3400, 3100, 3000-2800, 1650, 1450, 1400, 1250, 1090.

## Exampel 4

### 2-Hydroxy-4-(1-imidazolylmethyl)benzoic acid hydrobromide (I, R=R'= H. HBr, 4 position)

To 4,38 g of 4-(1-imidazolylmethyl)-2-methoxybenzoic acid in 190 ml of dry methylene chloride, under cooling to -75°C, 4,7 ml of boron tribromide are added. The mixture is stirred for 15 minutes at -75°C and further 24 hours at room temperature. The insoluble residue is filtered off, dried and recrystallized from ethanol to give 4,0 g (yield: 71 %).

IR Spectrum (KBr), $cm^{-1}$: 3400, 3200-2600, 1650, 1405, 1190, 1150, 800.

## Exampel 5

### Methyl 5-bromomethyl-2-methoxybenzoate (II, 5 position)

A mixture of 40,88 g of methyl 5-methyl-2-methoxybenzoate (B.P.: 80-85°C/0,2 torrs and $n_2^{20}$ = 1,5314), 40,37 g of N-bromosuccinimide and 0,5 g of benzoyl peroxide in 220 ml of carbon tetrachloride is refluxed for 3 hours. Then, the mixture is cooled, the succinimide is filtered off, the solvent is evaporated to dryness and the residual solid (M.P.: 77-80°C) corresponds to the desired product.

IR Spectrum (KBr), $cm^{-1}$: 1690, 1300, 1260, 1020.

## Example 6

**Methyl 5-(1-imidazolylmethyl)-2-methoxybenzoate**
**(I, R=R'= $CH_3$, 5 position)**

To 3, 14 g of sodium hydride in 290 ml of dry N,N-dimethylformamide are added dropwise 9,45 g of imidazole in 25 ml of N,N-dimethylformamide and the mixture is heated to 90°C for 1 hr. Then it is allowed to cool and 34,1 g of methyl 5-bromomethyl-2-methoxybenzoate in 30 ml of N,N-dimethylformamide over a period of 45 minutes are added. The mixture is kept at 90°C for 1 hour and the solvent is removed under vacuum, followed by addition of water and several extractions with methylene chloride. The residue is recrystallized from ethyl acetate-hexane to give 13,9 g (yield: 40 %) of a solid.

M.P.: 67-68°C.

IR-Spectrum (KBr), $cm^{-1}$: 3100, 1695, 1500, 1260, 1085.

## Example 7

**5-(1-Imidazolylmethyl)-2-methoxybenzoic acid**
**(I, R= $CH_3$, R' = H, 5 position)**

To a solution of 9,1 g of methyl 5-(1-imidazolylmethyl)-2-methoxybenzoate in 75 ml of ethanol is added 24 ml of 2N potassium hydroxide and the mixture is refluxed for 2 hours. The ethanol is evaporated, 20 ml of water are added and the mixture is then extracted with ether; the aqueous phase is acidified with glacial acetic acid, thus crystallizing the product by cooling. The

residue is washed with water and dried to give 8,1 g (yield: 95 %) of a white solid. M.P.: 193-195°C. Analysis correct.

IR Spectrum (KBr), $cm^{-1}$: 3420, 3120, 3000-2200, 1680, 1490, 1260, 1010, 820.

## Beispiel 8

**2-Hydroxy-5-(1-imidazolylmethyl)benzoic acid (I, R=R'= H, 5 position)**

To 3,77 g of 5-(1-imidazolylmethyl)-2-methoxybenzoic acid in 160 ml of dry methylene chloride, under cooling at -75°C, 4,02 g of boron tribromide are added. The mixture is stirred for 15 minutes at -76°C and further 24 hours at room temperature. The insoluble residue is filtered off (5,6 g), treated with water, adjusted to a basic pH with 20 % sodium hydroxide solution, extracted with ether, and the aqueous phase is acidified with glacial acetic acid. The insoluble residue is filtered off and dried to give 1,25 g (yield: 50%) of a white solid. M.P.: 256-258°C.

IR Spectrum (KBr), $cm^{-1}$: 3420, 3200-2400, 1675, 1490, 1310, 1240, 1060, 990.

Patent Claims

1. 2-Substituted benzoic acid imidazoles of the general formula (I):

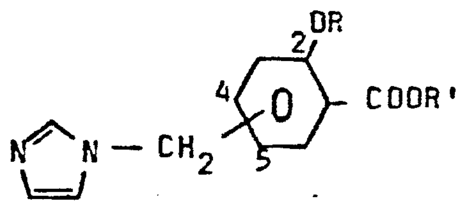

(I)

wherein

R and R' independently may be a hydrogen atom or a straight chain or branched $C_1$-$C_6$ alkyl group, and the imidazolylmethyl group is attached to the 4- or 5-position of the benzene ring,

and the non-toxic addition salts thereof.

2. The compounds according to claim 1, wherein R and R' are methyl.

3. The compounds according to claim 1, wherein R and R' are hydrogen.

4. The compounds according to claim 1, wherein R is methyl and R' is hydrogen.

5. Process for preparing the compounds according to claims 1 to 4, characterized in that

A) a compound of the general formula (II)

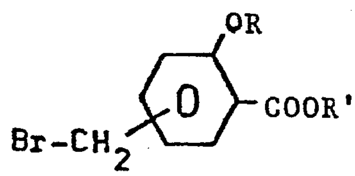

(II)

wherein R and R' have the meanings as defined in claim 1, and the bromomethyl group is attached to the 4- or 5-position of the benzene ring, is reacted with imidazole in an aprotic solvent in the presence of a strong base,

B) if desired, the so obtained ester of the general formula (Ia)

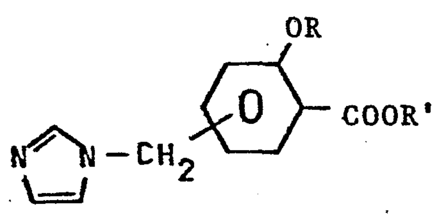

(Ia)

is saponified, and

C) if desired, the so obtained compound of the general formula (Ib)

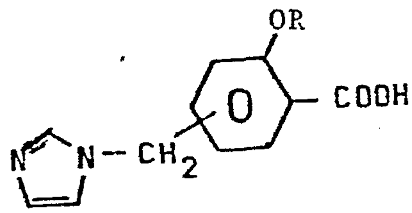

(Ib)

is subjected to an ether cleavage reaction, and

D) if desired, any of the compounds obtained above is converted to a non-toxic addition salt thereof.

6. The process according to claim 5, characterized in that in step A) the reaction is carried out in N,N-dimethylformamide in the presence of sodium hydride.

7. The process according to claim 5 characterized in that the ether cleavage of step C) is carried out with boron tribromide in a chlorinated aliphatic hydrocarbon.

8. Pharmaceutical composition comprising at least one of the compounds according to claims 1-4, optionally in combination with pharmaceutical carriers and/or adjuvants.